# FASCICULE DE BREVET EUROPEEN

(11) **EP 0 828 452 B1**
(45) Date de publication et mention de la délivrance du brevet: **25.06.2003**
(21) Numéro de dépôt: 97916503.2
(22) Date de dépôt: 26.03.1997
(51) Int. Cl.: A61B 5/117, G06K 9/00

(54) **DISPOSITIF CAPTEUR D'EMPREINTES**
FINGERABDRUCKSENSOR
FINGERPRINTING DEVICE

(30) Priorité: 28.03.1996 FR 9603881
(43) Date de publication de la demande: 18.03.1998
(73) Titulaire: SAGEM S.A., 75015 Paris (FR)
(72) Inventeur: CALMEL, Maryline, F-92370 Chaville (FR)
(74) Mandataire: Fort, Jacques
(86) Numéro de dépôt international: FR9700536
(87) Numéro de publication internationale: WO97036544

(56) Documents cités:
- EP-A- 0 359 554
- DE-A- 3 423 886
- US-A- 5 099 131
- US-A- 5 448 649

## Description

La présente invention concerne un dispositif capteur d'empreinte digitale, permettant de déceler les creux et les saillies d'une phalange.

Il existe déjà de tels dispositifs optiques tels que celui décrit dans le document FR-A-2 235 431, utilisant un réseau à deux dimensions de sites de détection, associé à un système de reconnaissance.

La présente invention vise à fournir un dispositif capteur d'empreintes digitales répondant mieux que ceux antérieurement connus aux exigences de la pratique, notamment du fait de sa simplicité et de son aptitude à bien différencier les creux et les saillies, donc à fournir une empreinte ayant des lignes nettes.

Dans ce but, l'invention propose un dispositif capteur d'empreinte digitale conforme à l'une quelconque des revendications 1 à 7.

Chacun des éléments constitutifs du dispositif peut prendre des formes très différentes.

Dans des modes avantageux de réalisation, les sources sont constituées par des diodes électro-luminescentes en couches minces déposées sur la face interne du substrat, par des barrettes parallèles de diodes électroluminescentes ou un composant plat continu. Un tel composant plat peut par exemple être formé par une plaque en matériau diffusant, telle qu'une résine acrylique, dont la face inférieure porte des diodes électroluminescentes dont le nombre peut être très inférieur à celui des éléments photo-sensibles, afin de réduire le coût du dispositif. Ces diodes électro-luminescentes ou LED peuvent être collées sur la face inférieure du support, éventuellement dans des cavités prévues à cet effet. Le substrat peut ainsi constituer un composant qui dévie ou diffuse, vers le support, de la lumière reçue de sources placées latéralement ou longitudinalement par rapport à lui. Le support, de quelques millimètres d'épaisseur, peut alors être traité pour constituer des micro-miroirs semi-transparents renvoyant la lumière vers les intervalles.

De façon générale, les sources peuvent être fixées sur la face externe ou la face interne du substrat ou sur une ou plusieurs des tranches de ce dernier.

Les éléments photosensibles peuvent eux aussi avoir diverses constitutions. Il peut s'agir de photodiodes intégrées dans une couche mince de silicium appartenant au support ou le constituant. Il peut s'agir de phototransistors en couche mince, similaires à ceux utilisés dans les écrans plats de visualisation. Il peut encore s'agir de cellules photovoltaïques.

Les sources utilisables disponibles à bas coût sont surtout des sources émettant dans le visible ; on utilisera généralement de telles sources et des éléments photosensibles qui leur sont appariés pour présenter une sensibilité élevée dans leur plage d'émission.

Les caractéristiques ci-dessus ainsi que d'autres apparaîtront mieux à la lecture de la description qui suit d'un mode particulier de réalisation de l'invention, donné à titre d'exemple non limitatif. La description se réfère aux dessins qui l'accompagnent, dans lesquels :
- la figure 1 est une vue d'ensemble en perspective montrant une constitution possible d'un dispositif capteur,
- la figure 2 est une vue en coupe schématique destinée à montrer le mode de différenciation des creux et des saillies lors de la prise d'empreinte par le capteur.

Le dispositif capteur qui sera décrit à titre d'exemple est associé à un système d'exploitation des signaux obtenus. Ce système peut être de l'un quelconque des types actuellement connus et par exemple du type décrit dans le document FR-A- 2 235 431, déjà mentionné, auquel on pourra se reporter.

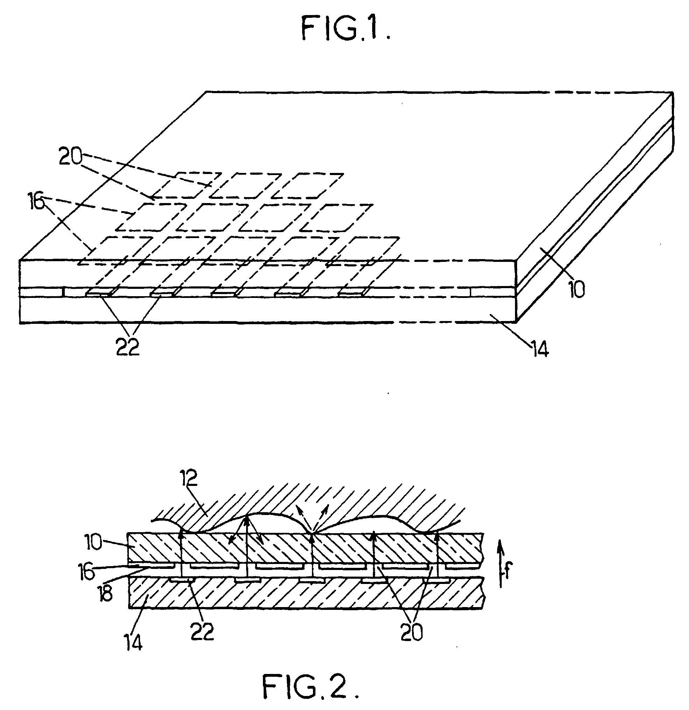

Le dispositif capteur montré schématiquement en figure 1 peut être regardé comme comportant un support transparent 10 sur lequel vient s'appuyer le doigt 12 pour obtenir une empreinte et un substrat 14 fixé au support 10.

La face interne du support 10 porte une matrice à deux dimensions d'éléments photosensibles 16, dont seuls quelques-uns sont représentés sur la figure 1. Ces éléments photosensibles sont protégés contre la lumière provenant du substrat (flèche f sur la figure 2), par exemple par un dépôt opaque 18. Ce dépôt peut être métallique. Son épaisseur doit être suffisante pour que sa transmission soit pratiquement nulle. Le dépôt peut être réfléchissant, ce qui permet de réduire les pertes de lumière.

Comme on l'a indiqué plus haut, les éléments photosensibles sont séparés par des intervalles 20 de passage de la lumière.

La constitution des éléments photosensibles 16 peut être l'une de celles couramment utilisées à l'heure actuelle lorsqu'une faible épaisseur est requise. On peut notamment utiliser des photodiodes ou phototransistors en couche mince réalisés sur un support 10 transparent, habituellement en verre ou en quartz.

Le pas de répartition des éléments 16 doit être plus faible que la distance séparant des creux successifs d'empreintes. Dans la pratique, ce pas ne doit pas dépasser 80 µm. Le pas peut être nettement plus faible, mais on ne gagne pas en sélectivité en descendant de 50 µm, de sorte que l'on adoptera en général un pas de répartition compris entre 50 et 80 µm.

Les signaux de sortie des éléments photosensibles sont transmis hors du dispositif capteur par des pistes étroites constituées de façon classique en technologie couche mince.

En particulier, les éléments photosensibles peuvent avoir une disposition matricielle à balayage ligne par ligne. Chaque élément photosensible comportera alors une zone de détection de lumière constituant un pixel particulier, associé à un circuit d'attaque, tel qu'un transistor en couche mince relié à la fois à un conducteur de colonne et à un conducteur de ligne. Les conducteurs et les raccordements peuvent être constitués par des pistes étroites, suffisamment minces pour être transparentes, ou même opaques si elles sont étroites, par exemple en or.

Le dispositif capteur comporte également des moyens destinés à transmettre, du substrat vers le capteur, de la lumière qui traverse les intervalles dans une direction aussi orthogonale que possible au support.

Dans le cas illustré sur la figure 1, ces moyens sont constitués par des bandes ou barrettes 22 photoémissives, ce qui facilite leur alimentation électrique. Il peut notamment s'agir de diodes électroluminescentes du genre utilisé dans les récepteurs de télécopie qui pourraient être déposées en couches minces sur le substrat. Ces diodes sont choisies de façon à correspondre au pic de sensibilité des éléments photosensibles, c'est-à-dire généralement à une longueur d'onde d'environ 640 manomètres. On peut même utiliser des barrettes parallèles.

Dans un autre mode de réalisation, les sources sont constituées par des diodes électroluminescentes individuelles placées chacune au croisement de deux intervalles. Ces diodes peuvent porter chacune une lentille de concentration du flux lumineux émis, de façon à fournir un lobe d'émission aussi étroit que possible et aussi directif que possible vers le support. Les moyens d'émission de lumière peuvent également être constitués par un composant unique recouvrant la face interne du substrat 14, dont certaines zones peuvent être masquées pour que l'émission de lumière ne s'effectue qu'à partir de portions situées en face des intervalles ou des croisements des intervalles. Le masquage peut s'effectuer par sérigraphie, peinture, dépôt d'une couche réfléchissante, collage d'une bande adhésive opaque.

Il est également possible d'utiliser un substrat diffusant qui reçoit de la lumière provenant d'émetteurs fixés sur la tranche ou fixés sur la face arrière. Dans ce cas encore, un masque peut être prévu sur la face interne du substrat.

Le mode de fonctionnement du dispositif capteur apparaît sur la figure 2. La lumière incident arrivant dans un creux est diffusée par ce creux et revient partiellement vers un ou plusieurs éléments photosensibles. Au contraire, la lumière incidente arrivant sur une saillie en contact avec le support est absorbée par le doigt. Les éléments sensibles situés en face ne reçoivent pratiquement pas de lumière. Les moyens de lecture (non représentés) associés aux éléments photosensibles peuvent être prévus pour comparer la tension ou le courant fournis par l'élément à un seuil de façon à assurer une différenciation.

Le dispositif capteur qui vient d'être décrit fournit une représentation directe de l'empreinte à l'échelle 1, ce qui est un avantage du point de vue de l'exploitation. Le dispositif capteur peut être réalisé par des technologies bien maîtrisées à l'heure actuelle de miniaturisation.

## Revendications

1. Dispositif capteur d'empreintes digitales, comprenant:
un support (10) transparent, plat et mince ayant une face externe destinée à recevoir l'extrémité d'un doigt et portant sur une face interne une matrice à deux dimensions d'éléments photosensibles (16) répartis suivant un pas régulier au plus égal à 90 µm, séparés par des intervalles (20) en bande transparents, de largeur inférieure à celle des éléments photosensibles ;
un substrat (14) portant, sur une face externe ou une face interne dirigée vers le support, des sources de lumière destinées à diriger, à travers les intervalles en bande du support, un faisceau de lumière traversant le support sensiblement orthogonalement à ladite face externe du support (10); et
des moyens d'alimentation desdites sources et de lecture des éléments photosensibles, lesdits éléments photosensibles étant protégés contre la lumière provenant des sources de façon à ne fournir un signal de sortie qu'en réponse à la lumière rétro-diffusée vers la face externe du support.

2. Dispositif selon la revendication 1, **caractérisé en ce que** les sources sont constituées par des diodes électro-luminescentes en couche mince déposées sur le substrat, des barrettes parallèles de diodes électro-luminescentes ou un composant plat continu.

3. Dispositif selon la revendication 2, **caractérisé en ce que** les sources sont constituées par des diodes électro-luminescentes individuelles portant chacune une lentille de concentration du flux lumineux émis.

4. Dispositif selon l'une quelconque des revendications précédentes, **caractérisé en ce que** les éléments photosensibles sont constitués par des photodiodes intégrées dans une couche mince de silicium constituant le support.

5. Dispositif selon l'une quelconque des revendications 1 à 4, **caractérisé en ce que** les éléments photosensibles sont des phototransistors en couches minces ou des cellules photovoltaïques.

6. Dispositif selon la revendication 4 ou 5, **caractérisé en ce que** les sources sont d'un type émettant dans l'infra-rouge proche ou dans le visible.

7. Dispositif selon la revendication 1, **caractérisé en ce que** le substrat est un composant de déviation vers le support de lumière reçue de sources placées latéralement ou longitudinalement par rapport au substrat.

## Patentansprüche

1. Fingerabdrucksensor, umfassend:
- einen flachen und dünnen transparenten Träger (10), der eine Außenseite besitzt, die dazu bestimmt ist, das Ende eines Fingers aufzunehmen, und auf einer Innenseite eine zweidimensionale Matrix von lichtempfindlichen Elementen (16) trägt, die in einem regelmäßigen Schritt von höchstens gleich 90 µm verteilt sind und durch bandförmige transparente Zwischenräume (20) getrennt sind, deren Breite kleiner als die der lichtempfindlichen Elemente ist,
- ein Substrat (14), das auf einer Außenseite oder einer Innenseite, die dem Träger zugewandt ist, Lichtquellen trägt, die dazu bestimmt sind, durch die bandförmigen Zwischenräume des Trägers hindurch ein Lichtbündel zu richten, das den Träger im wesentlichen rechtwinklig zu dieser Außenseite des Trägers (10) durchquert, und
- Mittel zur Versorgung dieser Quellen und zum Lesen der lichtempfindlichen Elemente, wobei diese lichtempfindlichen Elemente gegen das von den Quellen kommende Licht geschützt sind, so daß nur als Antwort auf das auf die Außenseite des Trägers zurückgestreute Licht ein Ausgangssignal geliefert wird.

2. Vorrichtung nach Anspruch 1, **dadurch gekennzeichnet, daß** die Quellen aus Dünnschicht-Elektrolumineszenzdioden, parallelen Leisten von Elektrolumineszenzdioden oder einem flachen durchgängigen Bauelement bestehen.

3. Vorrichtung nach Anspruch 2, **dadurch gekennzeichnet, daß** die Quellen aus einzelnen Elektrolumineszenzdioden bestehen, die jeweils eine Linse zur Konzentrierung des abgestrahlten Lichtflusses tragen.

4. Vorrichtung nach einem der vorhergehenden Ansprüche, **dadurch gekennzeichnet, daß** die lichtempfindlichen Elemente aus Photodioden bestehen, die in eine den Träger bildende dünne Siliciumschicht integriert sind.

5. Vorrichtung nach einem der Ansprüche 1 bis 4, **dadurch gekennzeichnet, daß** die lichtempfindlichen Elemente Dünnschicht-Phototransistoren oder Photoelemente sind.

6. Vorrichtung nach Anspruch 4 oder 5, **dadurch gekennzeichnet, daß** die Quellen von einem Typ sind, der im nahen Infrarot oder im sichtbaren Bereich sendet.

7. Vorrichtung nach Anspruch 1, **dadurch gekennzeichnet, daß** das Substrat ein Bauelement ist, das Licht, das von bezüglich des Substrats seitlich oder in Längsrichtung angeordneten Quellen empfangen wird, auf den Träger zu ablenkt.

## Claims

1. A fingerprint sensor device, comprising :
a thin flat transparent support (10) having an outside face designed to receive the end of a finger and carrying on an inside face a two-dimensional matrix of photosensitive elements (16) distributed at a regular pitch of not more than 90µm, the elements being separated by gaps (20) in the form of transparent strips of width that is smaller that of the photosensitive elements;
a substrate (14) carrying, on an external face or an inside face facing the support, light sources for directing light beams through the support via the strip-shaped gaps of the support, substantially orthogonally to said outside face of the support (10); and
means for powering said sources and for reading the photosensitive elements, said photosensitive elements being protected against the light coming from the sources so as to provide an output signal only in response to light back-scattered towards the outside face of the support.

2. A device according to claim 1, **characterized in that** the sources are constituted by thin-film light-emitting diodes deposited on the substrate, parallel strips of light emitting diodes, or a continuous flat component.

3. A device according to claim 2, **characterized in that** the sources are constituted by individual light emitting diodes, each carrying a lens for concentrating its emitted light flux.

4. A device according to any preceding claim, **characterized in that** the photosensitive elements are constituted by photodiodes integrated in a thin film of silicon constituting the support.

5. A device according to any one of claims 1 to 4, **characterized in that** the photosensitive elements are photocells or thin film phototransistors.

6. A device according to claim 4 or 5, **characterized in that** the sources are of a type that emits in the close infrared or in the visible.

7. A device according to claim 1, **characterized in that** the substrate is a component for deflecting towards the support light received from sources placed laterally or longitudinally relative to the substrate.
